(19) Europäisches Patentamt

European Patent Office

Office européen des brevets

(11) **EP 1 729 127 A1**

## (12) DEMANDE DE BREVET EUROPEEN

(43) Date de publication:
**06.12.2006 Bulletin 2006/49**

(51) Int Cl.:
***G01N 33/28*** (2006.01)

(21) Numéro de dépôt: **06290780.3**

(22) Date de dépôt: **11.05.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **02.06.2005 FR 0505683**

(71) Demandeur: **Institut Français du Pétrole 92852 Rueil-Malmaison Cedex (FR)**

(72) Inventeurs:
• **Schaeffer, Philippe**
  **67120 Emolsheim/Bruche (FR)**
• **Albrecht, Pierre**
  **67200 Strasbourg (FR)**
• **Rouquette, Nicolas**
  **76600 Le Havre (FR)**
• **Kowalewski, Isabelle**
  **78870 Bailly (FR)**
• **Fafet, Anne**
  **78420 Carrière-sur-Seine (FR)**

(54) **Méthode pour évaluer l'acidité d'échantillons pétroliers par marquage isotopique**

(57) - Elle comporte un marquage par un isotope d'un élément chimique présent dans au moins une fonction acide présente dans des échantillons pétroliers. On détermine ensuite l'enrichissement isotopique des échantillons par spectrométrie de masse à rapport isotopique (IRMS). On en déduit l'acidité des échantillons. Cette méthode constitue une solution alternative à la mesure du Total Acid Number (TAN). En particulier, elle représente la seule possibilité de mesure fiable de l'acidité sur des échantillons pétroliers disponibles en faible quantité.
- Applications à l'évaluation économique d'un champ de production d'hydrocarbures.
- Applications à la détermination de la gamme de carbone responsable de l'acidité d'un brut.
- Applications au suivi de l'évolution de la distribution en acides d'un site pollué par des hydrocarbures.

**Figure 1**

EP 1 729 127 A1

## Description

La présente invention concerne une méthode d'évaluation de l'acidité d'une huile.

En particulier, l'invention peut s'appliquer à tout échantillon pétrolier même en faible quantité (bruts, extraits, coupes, fractions pétrolières, ...), provenant d'un stade quelconque de l'industrie pétrolière : Exploration / Production / Raffinage / Environnement.

Sachant que la consommation mondiale énergétique sera encore assurée à 90% par les combustibles fossiles au cours des prochaines décennies, l'industrie pétrolière va être amenée de plus en plus à produire des huiles difficiles, en particulier des huiles lourdes. Ces huiles lourdes résultent, en majeure partie, de l'altération microbienne d'huiles conventionnelles, phénomène fondamental à l'origine des huiles acides. De ce fait, il s'agit donc d'un pôle important de renouvellement des réserves à moyen ou long terme, à condition de résoudre les problèmes techniques, économiques et environnementaux qui se posent pour les produire. En effet, ces bruts lourds biodégradés se caractérisent par une acidité importante (TAN >0,5) en plus de teneurs très fortes en métaux lourds, en soufre et en azote, teneurs bien supérieures à celles des huiles conventionnelles. Par conséquent, ces huiles nécessitent des procédés spécifiques pour la production, le transport et le traitement.

La présente invention prend tout son intérêt puisqu'elle va permettre d'évaluer l'acidité des bruts sur de faibles quantités en cours de production et/ou de transport à un stade précoce de caractérisation d'une formation souterraine et de sa mise en production. Elle va par ailleurs permettre de marquer sélectivement les molécules responsables de cette acidité au sein d'un échantillon pétrolier d'huile (brut ou extrait) afin de les identifier au mieux et d'appréhender ensuite leurs propriétés physico-chimiques.

## État de la technique

Les documents suivants que l'on va citer dans le cours de la description ci-après, illustrent l'état de la technique :

- Designation ASTM D664-95 : American Standard Test Method for Acid Number of Petroleum Products by potentiometric Titration, Annual Book of ASTM Standards, vol. 05.01., p.250-256

- Babaian-kibala, E., Petersen, P.R., Humphries, M.J., 1998. Corrosion by naphtenic acids in crude oils. Pre-prints of the A.C.S., Division of Petroleum Chemistry vol. 3, 106-110.

- Meredith, W., Kelland, S.-J., Jones, D.M., 2000. Influence of biodegradation on crude oil acidity and carboxylic acid composition. Org. Geochem. 31, 1059-1073.

- Robbins W.K., 1998, Challenges in the characterization of naphthenic acids in petroleum, 215th A CS Nat. Mtg. Dallas, preprints 43 (1), 137-140.

- Luo, Liwen; Xia, Daohong, 2003. Total carboxylic acids contents in petroleum reservoir rock determined by chemical titration. Prepr. - Am. Chem. Soc., Div. Pet. Chem. (Preprints - American Chemical Society, Division of Petroleum Chemistry) 48, 261-263

- Roussis s g; Lawlor 1 j, 2002. Direct determination of acid distributions in crudes and crude fractions. Patent assignee: Exxonmobil res & eng co world 02/48698a1, p 6/20/2002, f 11/6/2001, pr us 12/14/2000 (appl 60/255659) and us 9/21/2001 (appl 957941) (g01n-024/00)

L'acidité d'une huile est généralement définie dans l'industrie pétrolière à partir du nombre de milligramme de potasse alcoolique (KOH), nécessaire pour neutraliser un gramme d'huile. Ce nombre est appelé TAN pour "Total Acid Number". Ce TAN est obtenu par titration potentiométrique en milieu non aqueux. Cette titration s'effectue selon une méthode normée ASTM D664-95, décrite dans le document suivant :

American Standard Test Method for Acid Number of Petroleum Products by potentiometric Titration, Annual Book of ASTM Standards, vol. 05.01., p.250-256

Cette méthode potentiométrique présente l'inconvénient de nécessiter l'utilisation d'un grand volume de brut et par conséquent une quantité d'échantillons importante (Tableau 1). En effet, cette méthode se base sur la mesure d'une différence de potentiel électrique (exprimée en mV) entre un pétrole brut dilué dans un solvant (toluène/propanol-2) avant et après neutralisation référencée par rapport à une solution tampon acide anhydre fraîchement préparée. La mesure dépend de la sensibilité des électrodes qui doit mesurer de très faibles variations (échelle millivolt). C'est pourquoi, cette mesure requiert des quantités importantes d'huile ; plus l'acidité est faible, plus les quantités sont importantes (tableau 1). Par ailleurs, il est indispensable d'éliminer au préalable toute particule solide de l'échantillon pétrolier, avant d'appliquer cette méthode normée. Le calcul s'effectue selon la formule suivante :

$$TAN = \frac{m_{KOH}(mg)}{m_e(g)} = (A - B).M.\frac{56,1}{m_e(g)}$$

avec :

- $m_e(g)$ : masse de l'échantillon d'huile en g ;

- $m_{KOH}(mg)$ : masse de potasse alcoolique (KOH) en mg nécessaire à neutraliser la masse d'échantillon d'huile $m_e(g)$ ;

- B : volume en ml de KOH nécessaire à la titration du solvant en l'absence d'huile (test blanc) ;

- M : concentration de la solution de KOH en mole/l ;

- A : volume en ml de la solution de KOH (0,1 mole/l) nécessaire à la titration de l'huile diluée dans un solvant, jusqu'à la mesure d'un point d'inflexion la plus proche de celle obtenue pour la solution tampon.

NB : masse molaire de KOH = 15,9994 + 1,00797 + 39,103 = 56,1

Tableau 1: Quantité d'huile nécessaire pour en déterminer l'acidité d'après la norme D664-95

| TAN | Masse d'échantillon nécessaire (g) | Précision de la pesée (g) |
|---|---|---|
| 0,05-1,0 | 20,0±2,0 | 0,1 |
| 1,0-1,5 | 5,0±0,5 | 0,02 |
| 5-20 | 1,0±0,1 | 0,005 |
| 20-100 | 0,25±0,02 | 0,001 |
| 100-250 | 0,1±0,01 | 0,0005 |

Une huile est considérée comme acide si son TAN est supérieur à 0,5 mg de KOH par gramme d'huile. A partir des observations naturelles, l'échelle des TAN des bruts varie de 0,1 à 8 mg KOH/g huile (Babaian-Kibala *et al.,* 1998; Robbins *et al.,* 1998; Meredith et *al.,* 2000). Bien qu'une valeur de TAN égale à 0,5 soit faible, et que les composés acides restent toujours des constituants mineurs des huiles brutes, leur rôle peut être significatif en terme de rentabilité lors de l'exploitation d'un champ pétrolier (incertitude sur la qualité du réservoir, valeur économique faible du brut, ...). Ces acides sont, entre autres, responsables des problèmes d'émulsion, de moussage et de dépôts de savons (en cours de production), de corrosion (en cours de production, transport et/ou raffinage), et d'environnement (traitement des eaux, pollution de sites). Cette mesure d'acidité globale est à ce jour la seule utilisée et reconnue par les pétroliers.

Différentes méthodes connues portant sur la mesure de l'acidité des bruts et/ou la caractérisation des acides dans des huiles de production sont décrites par exemple dans les publication suivantes :

- Liwen, 2003, décrit une méthode de titration par retour (en excès de potasse) sur des carottes broyées testée en fonction de différents paramètres : taille de grains, temps d'extraction, volume de solvant (acide acétique/alcool), .... Les mesures de l'acidité (en mmoles/ g carotte) sont supérieures à la méthode normée et des conditions optimales sont précisées.

- Roussis : décrit une autre méthode pour la détermination directe de la distribution en acides dans des bruts ou des fractions de brut par spectrométrie de masse en mode d'ionisation chimique négative (Cl-). Les anions de chlorure générés par IC réagissent avec les composés acides des échantillons pétroliers. Les ions chlorés adduits sont détectés sélectivement et les espèces acides sont quantifiées à partir des pics identifiés.

De plus, les différentes méthodes proposées actuellement n'ont pas intégré de relation avec la valeur de TAN mesurée selon la norme ASTM D664 et ne sont donc pas utilisées par l'industrie pétrolière. Par ailleurs, ces analyses sont faites à un stade tardif de production, à partir d'huiles de stockage ou de test.

La méthode selon l'invention conduit à l'évaluation de l'acidité, traduite en terme d'enrichissement isotopique d'échantillons d'huile, même de faible quantité. La méthode est par ailleurs applicable à n'importe quel stade de l'industrie pétrolière (Exploration / Production / Raffinage / Environnement).

**La méthode selon l'invention**

L'invention concerne une méthode pour évaluer l'acidité d'un échantillon d'huile. Elle comporte les étapes suivantes :

- on réalise un enrichissement isotopique d'au moins une fonction acide présente dans ledit échantillon ;

- on détermine la valeur dudit enrichissement isotopique dudit échantillon ;

- on déduit l'acidité dudit échantillon à partir desdites valeurs d'enrichissement. Selon la méthode ledit enrichissement isotopique peut être réalisé à partir d'un des isotopes lourd suivants : $^{13}C$, $^{18}O$, D.

L'enrichissement peut comporter une conversion des fonctions acides carboxyliques en esters méthyliques à l'aide d'un réactif de méthylation marqué au $^{13}C$.

La détermination de la valeur d'enrichissement isotopique peut comporter les étapes suivantes:

- on mesure l'isotopie dudit élément chimique dudit échantillon avant enrichissement ;

- on mesure l'isotopie dudit élément chimique dudit échantillon après enrichissement ;

Les mesures de l'isotopie peuvent être réalisées par spectrométrie de masse isotopique.

Selon l'invention, on peut évaluer l'évolution de la biodégradation d'un site pollué à partir des étapes suivantes :

- on extrait dudit site des échantillons de roche contenant de la matière organique ;

- on extrait la matière organique de la matrice minérale ;

- on mesure des valeurs d'acidité $A_h$ de la matière organique à partir d'un enrichissement isotopique ;

- on évalue l'évolution de la biodégradation d'un site pollué à partir de l'acidité $A_h$.

Selon l'invention, on peut optimiser des conditions de production et de transport d'huile à partir des étapes suivantes :

- on extrait dudit site des échantillons de roche contenant de la matière organique ;

- on extrait la matière organique de la matrice minérale ;

- on mesure des valeurs d'acidité $A_h$ de la matière organique à partir d'un enrichissement isotopique ;

- on optimise les conditions de production et de transport de l'huile. Selon l'invention, on peut localiser et caractériser des molécules responsables de l'acidité, en appliquant les étapes suivantes à des extraits, des sous fractions d'huile ou des coupes d'huile:

- on mesure des valeurs d'acidité $A_h$ desdits extraits, sous fractions ou coupes à partir à partir d'un enrichissement isotopique ;

- on déduit desdites valeurs d'acidité $A_h$ pour chaque extraits, sous fractions ou coupes la localisation et la caractérisation des molécules responsables de l'acidité.

Les sous fractions d'échantillons d'huile peuvent être réalisées par chromatographie sur couche mince ou en phase liquide à partir de l'échantillon et les coupes d'échantillons d'huile par distillation.

Selon l'invention, on peut estimer une valeur de TAN à partir des étapes suivantes :

- on mesure des valeurs de TAN d'extraits de carottes de forage ;

- on mesure des valeurs d'acidité $A_h$ desdits extraits de carottes de forage à partir desdites valeurs d'enrichissement isotopique ;

- on détermine une loi permettant de relier lesdites valeurs de TAN aux dites valeurs d'acidité $A_h$ issues d'un enrichissement isotopique ;

- on estime une valeur de TAN à partir de ladite loi et à partir de toutes mesures valeurs d'acidité $A_h$ issues d'un enrichissement isotopique.

Enfin, selon l'invention, on peut estimer une valeur de TAN à partir d'une abaque construite à partir de valeurs d'acidité $A_h$ issues d'enrichissement isotopique.

**Présentation succincte des figures**

D'autres caractéristiques et avantages de la méthode selon l'invention, apparaîtront à la lecture de la description ci-après, en se référant aux figures ci-après annexées, parmi lesquelles :

- la figure 1 illustre la relation qui existe entre la mesure réalisée $\Delta\delta^{13}C$ *($A_h$)* et la valeur de TAN obtenue selon la norme ASTM D664 déterminées à partir d'huiles brutes de diverses origines géographiques et caractérisées par des propriétés physico-chimiques variées ;

- la figure 2 présente la relation qui existe entre la mesure réalisée $\Delta\delta^{13}C$ *($A_h$)* et l'altération (ALT) par biodégradation obtenues sur des extraits de carotte d'un champ pétrolier du Canada ;

- la figure 3 illustre la corrélation entre la valeur de TAN (norme ASTM D664) et $\Delta\delta^{13}C$ d'huiles africaines de même origine marquées au $^{13}C$.

**Description détaillée de la méthode**

Le principe de la méthode selon l'invention pour évaluer l'acidité d'échantillons pétroliers même disponibles en faible quantité, comporte un marquage isotopique des fonctions acides de ces échantillons. Elle peut être appliquée à n'importe quel stade de l'industrie pétrolière (Exploration / Production / Raffinage / Environnement).
La méthode peut alors se décomposer en trois étapes majeures :

1- on réalise un marquage isotopique des fonctions acides d'un échantillon d'huile ;

2- on mesure l'enrichissement en isotope de l'échantillon d'huile marqué et non marqué ;

3- on en déduit l'acidité de l'huile

1- Marquage isotopique des fonctions acides

Les composés responsables de l'acidité qui peuvent être présent dans un fluide pétrolier sont nombreux et principalement sous deux formes. On peut citer par exemple :

- les gaz acides : H2S et CO2 ;

- les acides organiques, phénols, ...

Cependant, l'acidité d'un fluide pétrolier, évaluée par son TAN, est essentiellement fonction de sa teneur en acides carboxyliques mesurée dans la fraction liquide. Ainsi, la fonction principale à enrichir par un isotope est la fonction acide carboxylique : RCOOH. Les isotopes stables utilisables par la méthode selon l'invention sont donc à rechercher parmi les isotopes du carbone, de l'oxygène et de l'hydrogène.
On peut citer par exemple le marquage des fonctions acides carboxyliques par de l'oxygène 18 ($^{18}O$), à l'aide

de $CH_3{}^{18}OH$ par exemple, puis d'utiliser le rapport ${}^{18}O/{}^{16}O$. On peut aussi utiliser l'isotope lourd de l'hydrogène, le deutérium à l'aide de $CD_3OH$, puis d'utiliser le rapport deutérium sur hydrogène *(D/H)*. Cependant, différents critères doivent être pris en compte pour obtenir une mesure rapide, précise, et peu chère :

- la quantité et le prix du réactif nécessaire au marquage, et contenant donc l'isotope stable, doivent être le plus faible possible ;

- le rapport entre l'isotope utilisé et l'isotope naturel doit être le plus élevé possible ;

- le réactif doit être facilement disponible (pas de réactions longues et compliquées, de produits rares, dangereux, ...) ;

- la réaction permettant la marquage des fonctions acides doit être simple dans sa mise en oeuvre ;

- le rendement de la réaction permettant la marquage des fonctions acides doit être satisfaisant.

C'est pourquoi, selon un mode de réalisation, on choisit de marquer les fonctions acides (et notamment les fonctions acides carboxyliques) par du carbone 13 ($^{13}C$). En effet le $^{13}C$ est facilement disponible et surtout le rapport $^{13}C/^{12}C$ est égal à 99,1%, ce qui est remarquable pour détecter de très faibles enrichissement en $^{13}C$. Le réactif utilisé peut par exemple être un alcool tel que le méthanol ou l'éthanol, le diazométhane ($CH_2N_2$) ou un halogènure. Dans le cas des halogènure, la réaction permettant l'enrichissement des fonctions acides s'écrit de la façon suivante :

$$ RCOO^- + {}^{13}CH_3{}_3X \longrightarrow RCOO{}^{13}CH_3 + X^- $$

Dans le réactif $^{13}CH_3X$, l'halogène X peut par exemple être le chlore ($CH_3Cl$), le brome ($CH_3Br$), ou l'iode ($CH_3I$). Le plus approprié et efficace (en terme de volatilité, réactivité et disponibilité commerciale) étant l'iodométhane, la méthode sera décrite à l'aide de ce réactif sans limiter l'invention.

Selon ce mode de réalisation, la méthode, simple de mise en oeuvre, consiste alors à convertir en milieu faiblement basique (en présence de carbonate de potassium $K_2CO_3$ pour former l'ion carboxylate $COO^-$) les fonctions acides carboxyliques présentes dans un échantillon pétrolier en esters méthyliques à l'aide d'un réactif de méthylation marqué au carbone 13 (équation 1) :

$$ RCOO^- + {}^{13}CH_3I \longrightarrow RCOO{}^{13}CH_3 + I^- \qquad (1) $$

Quelle que soit la nature de l'échantillon (huile, extrait, fraction, coupe d'huile, ...), la méthode de marquage est la même que celle décrite ci-après dans le cadre des huiles brutes. Cependant, la préparation de certains types d'échantillons nécessitera d'éventuelles étapes d'extraction et/ou de séparation/distillation préalables bien connues de l'homme du métier.

Selon un premier exemple d'application, on décrit l'application de la méthode à un volume d'huile brute. A 20 mg d'huile brute placés dans un ballon de 5 mL, sont ajoutés 20 mg de carbonate de potassium $K_2CO_3$ (formation de sel $COO-K^+$), 1 mL de tétrahydrofurane, THF (solvant de dissolution), 1 mL de méthanol (aide à la dissolution du carbonate de potassium pour former une solution homogène) et 50 $\mu$L d'iodométhane marqué au $^{13}C$ (Cambridge Isotope Laboratories, 99%). Le milieu réactionnel est ensuite placé sous reflux avec agitation magnétique, à 70°C, pendant 3 heures. Après retour à température ambiante, les solvants et l'excès d'iodométhane sont évaporés sous flux d'argon pendant 15 minutes, puis le brut réactionnel est extrait avec du dichlorométhane (x3) aux ultrasons. La solution obtenue est filtrée sur célite (cette dernière étant déposée dans un entonnoir dont le fond est bouché par de la laine de verre) et les solvants sont à nouveau évaporés sous pression réduite. Le brut réactionnel ainsi traité est dilué dans du dichlorométhane de façon à obtenir une solution à 20 mg.mL$^{-1}$ dont 10$\mu$L sont prélevés et déposés dans une nacelle en étain ou tout porte-échantillon nécessaire à l'analyse isotopique ultérieure. Après évaporation du solvant à température ambiante, la nacelle est fermée avec une pince puis déposée sur le plateau d'injection du passeur automatique de l'analyseur élémentaire couplé au spectromètre de masse isotopique.

Il faut noter qu'en effectuant un marquage selon cette méthode, d'autres fonctions peuvent être également touchées. En effet, la réaction de méthylation (équation 1) de l'ion carboxylate est une réaction de substitution nucléophile. Par conséquent, toute molécule possédant un doublet d'électrons libres (nucléophile) est susceptible de réagir selon ce mécanisme avec l'agent alkylant, comme par exemple les phénols ($C_6H_5OH$), les thiophénols ($C_6H_5SH$), les amines ($RNH_2$) ou les thiols ($RSH$). La spécificité de la réaction de marquage isotopique au carbone 13 vis à vis des acides carboxyliques a été vérifiée. Les tests réalisés sur des molécules modèles et sur des huiles riches en hétéroatomes (S, N, O) conclut que les acides carboxyliques ne sont pas les seules espèces pouvant être marquées dans les conditions opératoires utilisées. Les espèces fonctionnalisées comme les amines, les azaarènes, les phénols, les thiols et les sulfures peuvent réagir avec l'iodométhane, conduisant à la formation d'espèces marquées et à une surestimation de l'acidité de l'huile via la mesure $\Delta\delta^{13}C$. Plus la teneur en hétéroéléments (S, N) d'un brut sera importante et plus le taux d'incorporation de $^{13}C$ va être affecté sans qu'il s'agisse pour autant d'une huile à caractère acide important. Dans ce cas, la corrélation $\Delta\delta^{13}C$ - TAN n'est plus observée. Cependant, nos conditions expérimentales, qui sont relativement douces, limitent le nombre de réactions parasitaires évitant ainsi l'alkylation des espèces dont les valeurs de pKa sont supérieures à celles du carbonate de potassium ($K_2CO_3$).

<u>2- Mesure de l'enrichissement isotopique de l'échantillon d'huile</u>

Après enrichissement en carbone 13 des fonctions acides carboxyliques, on mesure l'isotopie du carbone (rapport $^{13}C/^{12}C$) de l'échantillon initial et de l'échantillon enrichi en $^{13}C$ puis on détermine l'enrichissement isotopique lié à l'incorporation de $^{13}C$. On réalise la mesure de l'isotopie du carbone par spectrométrie de masse isotopique (IRMS pour Isotope Ratio Mass Spectrometry). La mesure de l'enrichissement isotopique étant d'une très grande sensibilité, elle permet de mesurer un infime enrichissement en $^{13}C$, qui se traduit par une forte augmentation du rapport $^{13}C/^{12}C$ après marquage.
Les mesures isotopiques du carbone sont effectuées à l'aide d'un spectromètre de masse isotopique type Micromass Isoprime-EA utilisant un four de combustion type NC 2500 Thermoquest et un ordinateur Compaq Desktop EP series. Les conditions d'analyse isotopique sont les suivantes :

- Energie d'ionisation : 100 eV

- Température du réacteur de combustion :1030°C

- Température du réacteur de réduction : 650°C

- colonne chromatographique permettant de séparer ces différents gaz

- Débit d'hélium : 100 mL/min

<u>3- Évaluation de l'acidité de l'échantillon d'huile</u>

D'une part, l'incorporation de $^{13}C$ est proportionnelle à la quantité de fonctions acides (essentiellement carboxyliques) dans l'huile et d'autre part, l'enrichissement en $^{13}C$ se traduit par une augmentation du rapport $^{13}C/^{12}C$. Ainsi, la mesure de l'enrichissement isotopique, définie comme la différence entre la valeur isotopique du carbone 13 de l'huile de départ (non enrichie) et de l'huile enrichie avec du $^{13}C$, permet d'évaluer l'acidité $A_h$ de l'huile. On écrit ainsi la formule suivante :

$$A_h = \Delta\delta^{13}C = \delta^{13}C_e - \delta^{13}C_{ne}$$

avec :

- $A_h$ : acidité de l'huile

- $\Delta\delta^{13}C$ : enrichissement isotopique en carbone 13

- $\delta^{13}C_e$ : valeur isotopique du carbone 13 en ‰, de l'échantillon d'huile enrichi

- $\delta^{13}C_{ne}$ : valeur isotopique du carbone 13 en ‰, de l'échantillon d'huile non enrichi

Et l'on a pour un échantillon quelconque :

$$\delta^{13}C_{\text{échantillon}}(\%_{00}) = \frac{R_{\text{échantillon}} - R_{PDB}}{R_{PDB}} \times 1000$$

avec :

- $R$ : rapport du nombre d'atome de carbone 13 sur le nombre d'atome de carbone 12 : $R = {}^{13}C/{}^{12}C$

- $R_{PDB}$ : rapport $R$ correspondant à un standard international appelé "Pee Dee Belemnite".

$\Delta\delta^{13}C$ est donc proportionnel à l'acidité de l'huile et fournit donc une mesure de l'acidité $A_h$ qui est, comme décrit ultérieurement, proportionnelle à la valeur du TAN. Cette mesure est donc fiable et rapide. De plus elle peut être utilisée sur des échantillons de tout volume, même en très faible quantité, typiquement inférieure à 20mg. D'autres avantages et utilisations sont décrits ci-après.

4- Utilisations de la mesure isotopique de l'acidité d'un échantillon d'huile

*a) Corrélation avec les mesures de TAN*

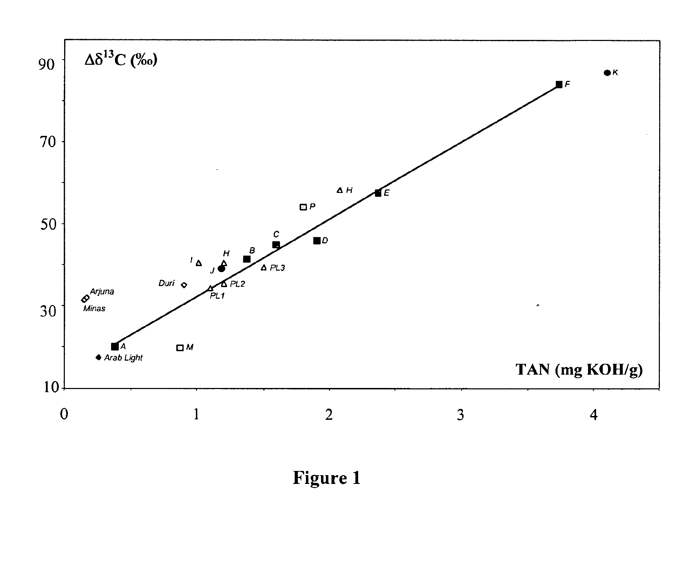

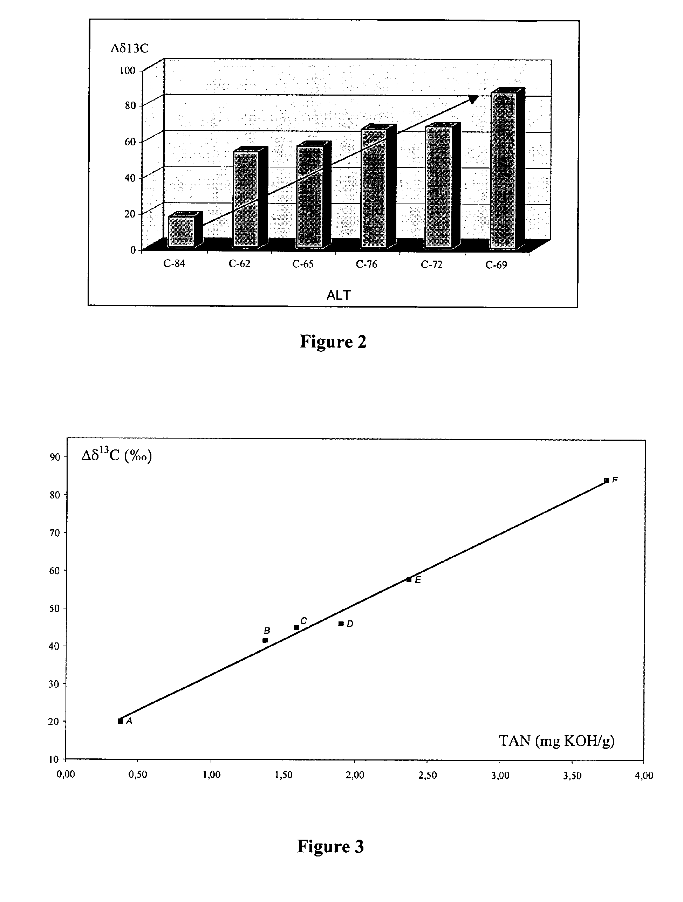

La méthode selon l'invention fournie une mesure de l'acidité à partir d'un marquage isotopique, qui peut être directement corrélé aux valeurs de TAN mesurées par la méthode normée ASTM D664. En effet, des mesures de l'acidité $A_h$ (à partir de l'enrichissement isotopique) sur des huiles brutes produites dans le monde entier montrent que dans l'ensemble, il existe une relation linéaire entre enrichissement isotopique et valeurs de TAN obtenues selon la méthode ASTM D664 comme illustrée sur la figure 1, qui montre l'acidité mesurée selon la méthode, à partir de l'enrichissement isotopique $\Delta\delta^{13}C$ (en ‰), en fonction du TAN pour ces différentes huiles brutes.
Ainsi, lors de l'exploration pétrolière et à partir des extraits de carottes de forage, il est possible d'estimer l'acidité de l'huile en place par voie conventionnelle, le TAN, et également grâce à la méthode d'enrichissement isotopique, l'acidité $A_h$. On peut alors à l'aide d'une régression estimer le TAN à partir des mesures de $A_h$. On obtient alors un TAN$_{\text{équivalent}}$. Ceci permet alors de pouvoir utiliser la méthode selon l'invention pour fournir une valeur d'acidité de l'huile qui peut être directement comparée au TAN par l'intermédiaire du TAN$_{\text{équivalent}}$. Selon la méthode on peut également fournir une abaque permettant de fournir directement une valeur de TAN à partir de la mesure de l'acidité $A_h$. Cette abaque est réalisée en effectuant sur des échantillons d'huile d'origines diverses des mesures de TAN et des mesures d'acidité $A_h$ selon l'invention.
Cette méthode de détermination de l'acidité a été appliquée à des huiles brutes (huiles notées de A à F) de même origine provenant d'un champ en Afrique en cours de production, et qui présentent des valeurs de TAN supérieures à 0,5mg KOH/g d'huile (déterminée au préalable selon la méthode normée ASTM D664). La figure 3 montre une parfaite régression linéaire entre l'enrichissement isotopique ($\Delta\delta^{13}C$ en ‰) et la mesure de TAN pour les échantillons A à F de degrés de biodégradation croissants.

*b) Localisation et caractérisation des molécules responsables de l'acidité*

La méthode de mesure de l'acidité à partir d'un marquage isotopique ne nécessite que de faibles quantités d'échantillons (<20 mg). Elle permet donc d'étudier les acides de sous fractions pétrolières (fractions chromatographiques, par exemple). La méthode permet ainsi de localiser et caractériser les molécules responsables de l'acidité dans les huiles.
On procède tout d'abord à l'obtention des fractions/coupes d'huiles ou extraits :

*\* Fractions d'huiles ou d'extraits*

Des fractions d'huiles ou d'extraits de différentes classes de polarité et/ou de différentes familles chimiques sont obtenues par chromatographie sur couche mince ou en phase liquide à partir de l'échantillon. On obtient alors la répartition en pour-cent poids entre chacune de ces familles chimiques.

*\* Coupe d'huiles ou d'extraits*

Les coupes d'huiles *ou d'extraits* sont obtenues par distillation. La gamme de carbone de chaque coupe est déterminée en se basant sur les températures d'ébullition de point initial et final de la coupe dans les conditions de pression de la distillation. On obtient alors la répartition en pour-cent poids entre chacune des coupes réalisées.

Puis le protocole de marquage isotopique décrit précédemment est appliqué sur environ 20 mg des différentes fractions/coupes obtenues pour déterminer l'enrichissement isotopique de chacune d'elles, et donc de caractériser les molécules responsables de l'acidité dans les huiles ou les extraits. La mesure de l'acidité obtenue par la méthode est plus précise que les méthodes antérieures puisque la mesure peut être effectuée sur des fractions ou coupes d'échantillons et ainsi conduire à localiser les molécules responsables de l'acidité

*c) Aide à l'évaluation technico-économique d'un champ pétrolier*

La production d'huiles acides posent de nombreux problèmes techniques, économiques et environnementaux. En effet, ces bruts lourds biodégradés se caractérisent par une acidité importante (TAN >0,5) en plus de teneurs très fortes en métaux lourds, en soufre et en azote, teneurs bien supérieures à celles des huiles conventionnelles. Par conséquent, ces huiles nécessitent des procédés spécifiques pour la production, le transport et le traitement.

Il est donc très important de pouvoir définir l'acidité d'une huile le plus tôt possible dans l'exploitation d'un champ pétrolier. Or, l'évaluation du TAN nécessite, on l'a vu, de grande quantité d'huile. En revanche, la méthode selon l'invention fournit une mesure sur de faibles quantités. Ainsi, à un stade précoce de production, bien que l'on ait très peu d'huile, il est possible d'évaluer l'acidité $A_h$. A partir de cette valeur et éventuellement par estimation d'un équivalent TAN, TAN$_{équivalent,}$ il est possible de connaître rapidement les conditions optimales de production, de transport et de traitement de l'huile à produire.

A partir de la méthode de mesure de l'acidité selon l'invention, il est possible d'évaluer ces conditions optimales. Pour ce faire, on travaille sur des échantillons de roche, tels que des carottes, des "side wall cores", des déblais... Ensuite, on extrait la matière organique de la matrice minérale par une technique d'extraction par solvant. L'extraction quantitative, de la matière organique contenue dans une roche broyée (à partir de 1 g) est réalisée avec le dichlorométhane utilisé comme solvant d'extraction et dans une proportion minimum de 10 ml par gramme de roche pendant 1 heure à reflux à 40°C. Après retour à température ambiante, la roche plus l'extrait sont filtrés sous vide. L'extrait recueilli dans un ballon est concentré à l'évaporateur puis transféré dans une nacelle d'aluminium pour y être pesé afin d'obtenir la quantité d'extrait contenu dans la roche.

La figure 2 présente l'enrichissement isotopique du carbone 13 ($\Delta\delta^{13}C$ en ‰) obtenu pour des extraits C$_{14+}$ de carottes canadiennes altérés à différents degrés de biodégradation (ALT) qui proviennent d'un même champ et dont la source est unique. L'intensité du marquage isotopique est corrélée avec l'intensité de la biodégradation des extraits de carottes déterminée sur la base de l'analyse des marqueurs moléculaires présents dans les différentes extraits C$_{14+}$. La méthode selon l'invention permet donc de déterminer l'acidité d'une huile d'un champ pétrolier à un stade précoce d'exploitation, permettant ainsi de réaliser une évaluation technico-économique d'un champ pétrolier à un stade plus précoce qu'avec une mesure de TAN.

*d) Évolution de la biodégradation d'un site pollué*

A partir de la méthode de mesure de l'acidité selon l'invention, il est possible d'évaluer l'évolution de la biodégradation d'un site pollué. Pour ce faire, on travaille sur des échantillons de roche tels que des carottes. Ensuite, on extrait la matière organique de la matrice minérale par une technique d'extraction connue.

On peut reprendre l'exemple de la figure 2 qui présente l'acidité obtenu pour des extraits C$_{14+}$ de carottes canadiennes d'un même champ, altérés à différents degrés de biodégradation. On constate que l'intensité du marquage isotopique (ou $A_h$) est corrélé avec l'intensité de la biodégradation des extraits de carottes déterminée sur la base de l'analyse des marqueurs moléculaires présents dans les différentes extraits C$_{14+}$.

De cette façon, il est également possible de suivre l'évolution de l'acidité (corrélé à l'enrichissement isotopique) d'un site pollué, l'augmentation de l'enrichissement isotopique allant de pair avec l'augmentation de l'intensité de la biodégradation.

Les avantages de la méthode apparaissent alors nettement :

- la méthode de l'enrichissement isotopique en carbone 13 des extraits de carottes de forage permet, lors de l'exploration pétrolière, une estimation de l'acidité de l'huile en place, qui peut être directement corrélée aux valeurs de TAN par le calcul d'un équivalent TAN.

- la mesure de l'acidité selon l'invention peut être faite à un stade très précoce (avant production), ce qui permet une évaluation technico-économique beaucoup plus fiable. Les moyens à mettre en oeuvre pour la production, le transport et les procédés de raffinage peuvent être optimisés (choix d'additifs, de catalyseurs, de gaines de flexibles,...).

- la mesure de l'acidité selon l'invention permet de suivre facilement l'évolution de la biodégradation sur un site pollué à partir d'échantillons de carottes par exemple.

- la prévision obtenue selon la méthode de marquage isotopique est plus précise, puisque la mesure peut être effectuée sur des fractions ou coupes d'échantillons et ainsi conduire à localiser les molécules responsables de l'acidité

**Revendications**

1. Méthode pour évaluer l'acidité d'un échantillon d'huile, **caractérisée en ce qu'**elle comporte les étapes suivantes :

   - on réalise un enrichissement isotopique d'au moins une fonction acide présente dans ledit échantillon ;
   - on détermine la valeur dudit enrichissement isotopique dudit échantillon ;
   - on déduit l'acidité dudit échantillon à partir desdites valeurs d'enrichissement.

2. Méthode selon la revendication 1, dans laquelle ledit enrichissement isotopique est réalisé à partir d'un des isotopes lourd suivants : $^{13}$C, $^{18}$O, D.

3. Méthode selon la revendication 2, dans laquelle ledit isotope est du carbone 13 ($^{13}$C).

4. Méthode selon la revendication 3, dans laquelle ledit enrichissement comporte une conversion des fonctions acides carboxyliques en esters méthyliques à l'aide d'un réactif de méthylation marqué au $^{13}$C.

5. Méthode selon l'une des revendications précédentes, dans laquelle la détermination de la valeur d'enrichissement isotopique comporte les étapes suivantes :

   - on mesure l'isotopie dudit élément chimique dudit échantillon avant enrichissement ;
   - on mesure l'isotopie dudit élément chimique dudit échantillon après enrichissement ;

6. Méthode selon la revendication 5, dans laquelle lesdites mesures sont réalisées par spectrométrie de masse isotopique.

7. Méthode selon l'une des revendications précédentes, dans laquelle on évalue l'évolution de la biodégradation d'un site pollué à partir des étapes suivantes :

   - on extrait dudit site des échantillons de roche contenant de la matière organique ;
   - on extrait la matière organique ;
   - on mesure des valeurs d'acidité $A_h$ de la matière organique à partir d'un enrichissement isotopique ;
   - on évalue l'évolution de la biodégradation d'un site pollué à partir de l'acidité $A_h$.

8. Méthode selon l'une des revendications 1 à 6, dans laquelle on optimise des conditions de production et de transport d'huile d'un champ pétrolier à partir des étapes suivantes :

   - on extrait dudit champ des échantillons de roche contenant de la matière organique ;
   - on extrait la matière organique ;
   - on mesure des valeurs d'acidité $A_h$ de la matière organique à partir d'un enrichissement isotopique ;
   - on optimise les conditions de production et de transport de l'huile à partir de l'acidité $A_h$.

9. Méthode selon l'une des revendications 1 à 6, dans laquelle on localise et l'on caractérise des molécules responsables de l'acidité, en appliquant les étapes suivantes à des extraits, des sous fractions d'huile ou des coupes d'huile:

   - on mesure des valeurs d'acidité $A_h$ desdits extraits, sous fractions ou coupes à partir d'un enrichissement isotopique ;
   - on déduit desdites valeurs d'acidité $A_h$ pour chaque extraits, sous fractions ou coupes la localisation et la caractérisation des molécules responsables de l'acidité.

10. Méthode selon la revendication 9, dans laquelle on réalise des sous fractions d'échantillons d'huile par chromatographie sur couche mince ou en phase liquide à partir de l'échantillon.

11. Méthode selon l'une des revendications 9 et 10, dans laquelle on réalise des coupes d'échantillons d'huile par distillation.

12. Méthode selon l'une des revendications 1 à 6, dans laquelle on estime une valeur de TAN à partir des étapes suivantes :

   - on mesure des valeurs de TAN d'extraits de carottes de forage ;
   - on mesure des valeurs d'acidité $A_h$ desdits extraits de carottes de forage à partir d'un enrichissement isotopique ;
   - on détermine une loi permettant de relier lesdites valeurs de TAN aux dites valeurs d'acidité $A_h$ ;
   - on estime une valeur de TAN à partir de ladite loi et à partir de toutes mesures d'acidité $A_h$ issues d'un enrichissement isotopique.

13. Méthode selon l'une des revendications 1 à 6, dans laquelle on estime une valeur de TAN à partir d'une abaque construite à partir de valeurs d'acidité $A_h$ issues d'enrichissement isotopique.

Figure 1

**Figure 2**

**Figure 3**

# EP 1 729 127 A1

**Office européen
des brevets**

**RAPPORT DE RECHERCHE EUROPEENNE**

Numéro de la demande

EP 06 29 0780

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,A | US 2002/086434 A1 (ROUSSIS STILIANOS G ET AL) 4 juillet 2002 (2002-07-04) <br> * alinéas [0006] - [0014], [0029] * <br> ----- | 1-13 | INV. <br> G01N33/28 |
| A | US 4 755 469 A (SHOWALTER ET AL) 5 juillet 1988 (1988-07-05) <br> * colonne 1, ligne 58-66 * <br> * colonne 2, ligne 21-41 * <br> ----- | 1-13 | |
| A | US 6 098 423 A (LEBLOND ET AL) 8 août 2000 (2000-08-08) <br> * colonne 3, ligne 22-24; figure 2 * <br> ----- | 1,6 | |
| A | EP 0 306 333 A (VG INSTRUMENTS GROUP LIMITED; FISONS PLC) 8 mars 1989 (1989-03-08) <br> * abrégé * <br> ----- | 1 | |
| | | | DOMAINES TECHNIQUES RECHERCHES (IPC) <br><br> G01N |

Le présent rapport a été établi pour toutes les revendications

| Lieu de la recherche | Date d'achèvement de la recherche | Examinateur |
|---|---|---|
| La Haye | 19 juillet 2006 | Wulveryck, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...........................................................
& : membre de la même famille, document correspondant

EPO FORM 1503 03.82 (P04C02)

14

**ANNEXE AU RAPPORT DE RECHERCHE EUROPEENNE
RELATIF A LA DEMANDE DE BREVET EUROPEEN NO.**

EP 06 29 0780

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-07-2006

| Document brevet cité au rapport de recherche | | Date de publication | Membre(s) de la famille de brevet(s) | | Date de publication |
|---|---|---|---|---|---|
| US 2002086434 | A1 | 04-07-2002 | AU | 3646702 A | 24-06-2002 |
| | | | CA | 2428901 A1 | 20-06-2002 |
| | | | EP | 1342074 A1 | 10-09-2003 |
| | | | JP | 2004515781 T | 27-05-2004 |
| | | | NO | 20032635 A | 11-06-2003 |
| | | | WO | 0248698 A1 | 20-06-2002 |
| US 4755469 | A | 05-07-1988 | AUCUN | | |
| US 6098423 | A | 08-08-2000 | BR | 9803467 A | 14-12-1999 |
| | | | DE | 19842646 A1 | 06-05-1999 |
| | | | FR | 2768512 A1 | 19-03-1999 |
| | | | GB | 2329468 A | 24-03-1999 |
| | | | NO | 984286 A | 19-03-1999 |
| EP 0306333 | A | 08-03-1989 | DE | 3855341 D1 | 11-07-1996 |
| | | | DE | 3855341 T2 | 10-10-1996 |
| | | | JP | 1127954 A | 19-05-1989 |
| | | | JP | 2762359 B2 | 04-06-1998 |
| | | | US | 4866270 A | 12-09-1989 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Designation ASTM D664-95 : American Standard Test Method for Acid Number of Petroleum Products by potentiometric Titration. Annual Book of ASTM Standards. vol. 05.01., 250-256
- **BABAIAN-KIBALA, E. ; PETERSEN, P.R. ; HUMPHRIES, M.J.** Corrosion by naphtenic acids in crude oils. *Pre-prints of the A.C.S., Division of Petroleum Chemistry,* 1998, vol. 3, 106-110
- **MEREDITH, W. ; KELLAND, S.-J. ; JONES, D.M.** Influence of biodegradation on crude oil acidity and carboxylic acid composition. *Org. Geochem.,* 2000, vol. 31, 1059-1073
- **ROBBINS W.K.** Challenges in the characterization of naphthenic acids in petroleum. *215th A CS Nat. Mtg. Dallas,* 1998, vol. 43 (1), 137-140
- **LUO, LIWEN ; XIA, DAOHONG.** Total carboxylic acids contents in petroleum reservoir rock determined by chemical titration. Prepr. *Am. Chem. Soc., Div. Pet. Chem.,* 2003, vol. 48, 261-263
- **ROUSSIS S G ; LAWLOR 1 J.** Direct determination of acid distributions in crudes and crude fractions. Patent assignee. *Exxonmobil res & eng co world 02/48698a1, p 6/20/2002, f 11/6/2001, pr us 12/14/2000 (appl 60/255659) and us 9/21/2001 (appl 957941) (g01n-024/00,* 2002
- American Standard Test Method for Acid Number of Petroleum Products by potentiometric Titration. Annual Book of ASTM Standards. vol. 05.01., 250-256